# EUROPEAN PATENT APPLICATION

(11) **EP 4 417 612 A1**
(43) Date of publication of application: **21.08.2024**
(21) Application number: 22857940.5
(22) Date of filing: 13.10.2022
(51) Int. Cl.: C07D 498/22, A61K 41/00, A61P 35/00

(54) **DEGRADABLE NEAR-INFRARED PHOTOSENSITIZER, AND PREPARATION METHOD THEREFOR AND USE THEREOF**

(30) Priority: 14.10.2021 CN 202111196449
(71) Applicant: Beijing Institute of Collaborative Innovation, Beijing 100094 (CN); Guangdong GBA Institute of Collaborative Innovation, Guangzhou, Guangdong 510700 (CN)
(72) Inventor: ZHANG, Junlong, Beijing 100094 (CN); ZHU, Mengliang, Beijing 100094 (CN); ZHANG, Hang, Beijing 100094 (CN); YANG, Zishu, Beijing 100094 (CN); WANG, Bingwu, Beijing 100094 (CN)
(74) Representative: Zaboliene, Reda
(86) International application number: PCT/CN2022/124995
(87) International publication number: WO 2023/020631

(57) **Abstract**

The present disclosure provides a degradable near-infrared photosensitizer, a preparation method, and an application thereof, and relates to the technical field of photosensitizer. The degradable near-infrared photosensitizer may be any one of a porphyrin compound, and a pharmaceutically acceptable salt, a solvate, a non-covalent bonded composite, a complex, and a prodrug of the porphyrin compound, and a structure of the porphyrin compound may be as shown in formula 1:

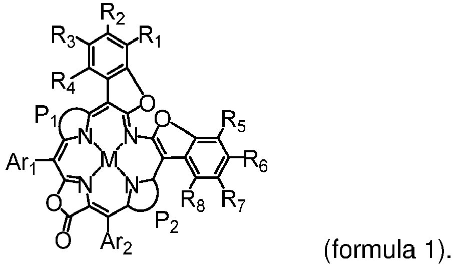

The present disclosure further provides a preparation method for the degradable near-infrared photosensitizer and an application of the degradable near-infrared photosensitizer. The degradable near-infrared photosensitizer provided by the present disclosure may be degraded while generating reactive oxygen species during irradiation, and when the irradiation is ended, the photosensitizer may be completely converted into a low-toxicity substance that does not have the photoactivity such that effectively solves a problem of a residue of the photosensitizer in the prior art.

## Description

### TECHNICAL FIELD

The present disclosure relates to the technical field of photosensitizers, and in particular, to degradable near-infrared photosensitizers, preparation methods, and applications thereof.

### BACKGROUND

Cancer is one of the diseases that seriously threaten human health, with a very high mortality rate, and has become the "number one killer" jeopardizing human life. Compared with the current main methods of cancer therapy, such as surgical resection, radiation therapy, and chemotherapy, etc., photodynamic therapy (PDT), as a non-invasive therapy method, includes advantages in therapeutic time, efficacy, drug resistance, toxicity, etc., and illustrates the great potential in the fields of cancer, oncology, dermatology, and antimicrobial therapy. The mechanism of photodynamic therapy includes injecting a photosensitizer into a living body, accumulating the photosensitizer at a lesion after arriving at the lesion through blood circulation, locally irradiating a lesion region with the light of a specific wavelength, exciting the photosensitizer, releasing reactive oxygen species (ROS), and killing lesion cells through direct damage, damaging lesion tissue blood vessels, inducing the immune stress, etc., to achieve the purpose of therapy. According to different mechanisms of reactive oxygen species generation, photodynamic therapy is able to be divided into type I or type II. A photosensitizer transfers and generates free radicals via electron or proton, a highly active free radical reacts with molecular oxygen to generate superoxide anion, and further reacts to generate active oxygen such as hydroxyl free radical and hydrogen peroxide, which is a type I photodynamic reaction, and a damage caused thereby is referred to as type I damage. The photosensitizer transfers excited state energy to surrounding oxygen molecules to convert the oxygen molecules into singlet oxygen, which is a type II photodynamic reaction, and damage caused thereby is referred to as type II damage.

The effectiveness of photodynamic therapy depends mainly on the nature of the photosensitizer. The photosensitizer should include strong phototoxicity and have no toxic side effects on the body. In recent years, the variety of photosensitizers has gradually increased, and there have been three generations of photosensitizers so far. A first generation of photosensitizer is mainly porphyrin mixtures. A second generation of photosensitizer refers to porphyrin derivatives with different structural types of monomers and synthetic compounds connected to the active molecules. A third generation of photosensitizer refers to a photosensitizer with a targeting function obtained by linking the photosensitizer to a monoclonal antibody or other small biologically active molecules such as steroids, lipids, peptides, nucleosides, and nucleotides. However, the scope of clinical application of photosensitizers is still limited, mainly because that: (1) a majority of photosensitizers still require short-wave excitation, and a penetration depth of short-wavelength light is insufficient, which makes it difficult to effectively irradiate a deep lesion; (2) the photosensitizer highly depends on oxygen, which leads to the limited therapeutic efficacy of photosensitizer for anoxic solid tumors; (3) a solubility is poor, which is prone to aggregate the photosensitizer in vivo and lacks specificity; and (4) a structure of the photosensitizer is stable, and the photosensitizer is slowly degraded and metabolized in vivo.

Because the conventional photosensitizer includes disadvantages such as a limited therapeutic depth, a high oxygen dependence, and a poor tumor targeting, an efficacy of photodynamic therapy in clinical applications is severely limited. In addition, after receiving photodynamic therapy, the residual photosensitizer in the body often takes a long time to be fully metabolized. Once the residual photosensitizer is exposed to irradiation, the residual photosensitizer will continue to produce reactive oxygen species and exhibit strong phototoxicity, thereby causing different degrees of damage to tissues and cells, forcing patients to avoid irradiation for a long period of time after receiving the therapy, and probably causing the patients to continue living in darkness and fear for months, which seriously affects physical health and mental health of the patients. Therefore, how to make the photosensitizer fail in time after an implementation of the photodynamic therapy such that avoid the safety problems caused by the residual photosensitizer has always been a difficult point in a clinical application of photodynamic therapy.

Therefore, it is desirable to provide a new degradable near-infrared photosensitizer, a preparation method, and an application thereof, in order to make up for the deficiencies of the prior art.

### SUMMARY

According to the embodiments of the present disclosure, one of the purposes of the present disclosure is to provide a degradable near-infrared photosensitizer.

The technical solution of the present disclosure for solving the above technical problem is as follows: a degradable near-infrared photosensitizer may be any one of a porphyrin compound, and a pharmaceutically acceptable salt, a solvate, a non-covalent bonded composite, a complex, and a prodrug of the porphyrin compound, and a structure of the porphyrin compound may be as shown in formula 1: 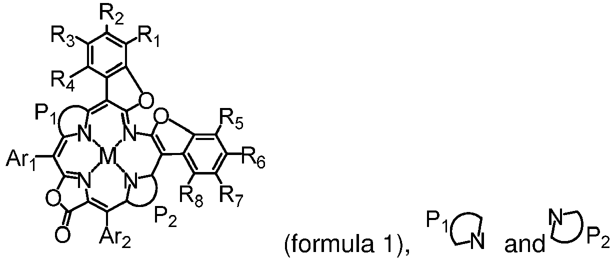 are independently selected from any one of 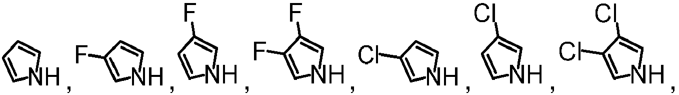 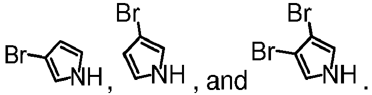 Ari and Ar₂ are substituted or unsubstituted phenyl. R₁, R₂, R₃, R₄, R₅, R₆, R₇, and R₈ may be independently selected from any one of hydrogen, halogen, nitro, hydroxyl, amino, hydroxy, carboxy, carboxylic, sulfonic, phosphoric acid, cyano, amido, C₁-C₈ alkyl substituted amino, substituted or unsubstituted C₁-C₈ alkyl, substituted or unsubstituted C₁-C₈ alkoxy, substituted thio, C₁-C₈ alkyl phosphoryl, C1-Cs alkyl carboxy, C₁-C₈ alkyl sulfonyl, C₃-C₆ alkylene, C₂-C₆ alkenyl, C₂-C₆ alkinyl, C₂-C₆ alkynyl, C₂-C₆ alkenyloxy, and C₂-C₆ alkynyloxy, and M may be a metal ion selected from any one of Mg, Cr, Mn, Fe, Co, Ni, Cu, Zn, Cd, Ga, Al, Pd, and Pt.

The description of the principle of the degradable near-infrared photosensitizer of the present disclosure may include:

The present disclosure may modify the structure of porphyrin compounds to adjust a photophysical property and a photochemical property of the compounds, realizing a near-infrared absorption of the photosensitizer, an emission property of the photosensitizer molecules, a type I photodynamic therapeutic capability and a type II photodynamic therapeutic capability. A lactone unit in the molecular structure of the photosensitizer may react with reactive oxygen species, when undergoing an irradiation excitation, the lactone unit may be converted into a low-toxicity substance that does not have a photoactivity and the photosensitizer may gradually degrade.

The beneficial effect of the degradable near-infrared photosensitizer of the present disclosure includes that: (1) the degradable near-infrared photosensitizer provided by the present disclosure is degraded while generating the reactive oxygen species during irradiation, and when the irradiation is ended, the photosensitizer is completely converted into a low-toxicity substance that does not have the photoactivity such that effectively solves a problem of a residue of the photosensitizer in the prior art. (2) an absorption spectrum of the degradable near-infrared photosensitizer provided by the present disclosure is in a near-infrared region (700 nm - 900 nm) with a large extinction coefficient, and an excitation light includes a greater tissue penetration depth, which helps to reduce a damage of a light source to the tissue. (3) The degradable near-infrared photosensitizer of the present disclosure includes a relatively strong fluorescence configured for fluorescence labeling and real-time monitoring of a degree of degradation of the photosensitizer. (4) The degradable near-infrared photosensitizer of the present disclosure illustrates a relatively high phototoxicity at both cellular and in vivo levels and is configured to prepare a photodynamic therapy medicine as an active ingredient.

Based on the above technical solution, the present disclosure may be improved as follows.

In some embodiments, Ari and Ar₂ may be independent 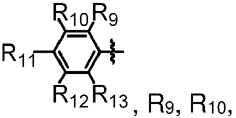 R₁₁, R₁₂ and R₁₃ may be independently selected from any one of hydrogen, halogen, nitro, hydroxyl, amino, hydroxy, carboxy, carboxylic, sulfonic, phosphoric acid, cyano, amido, C₁-C₈ alkyl substituted amino, substituted or unsubstituted C₁-C₈ alkyl, substituted or unsubstituted C₁-C₈ alkoxy, substituted thio, C₁-C₈ alkyl phosphoryl, C₁-C₈ alkyl carboxy, C₁-C₈ alkyl sulfonyl, C₃-C₆ alkylene, C₂-C₆ alkenyl, C₂-C₆ alkinyl, C₂-C₆ alkynyl, C₂-C₆ alkenyloxy, and C₂-C₆ alkynyloxy.

The beneficial effect of using the above embodiments includes: realizing a biocompatibility modification and a variation of function by adjusting a solubility and an activity of the photosensitizer through different substituent groups to adapt to different application requirements.

In some embodiments, a metal ion may be any one of Mg, Zn, Cd, Al, and Ga.

The beneficial effects of using the above embodiments include: adjusting an ability of the photosensitizer to produce fluorescence and active oxygen; improving a stability of the photosensitizer, further reducing a dark toxicity of the photosensitizer, and improving a biosafety.

In some embodiments, a specific structure of the porphyrin compound may be any one of:

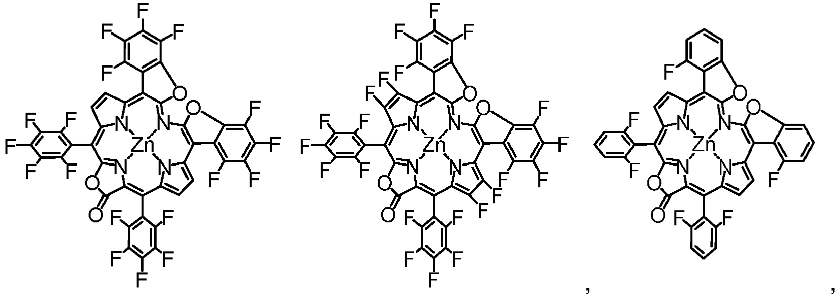

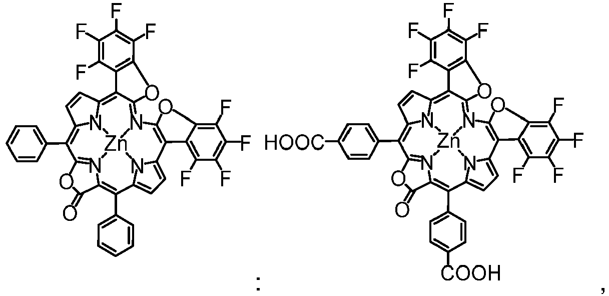

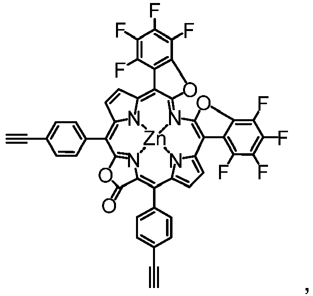

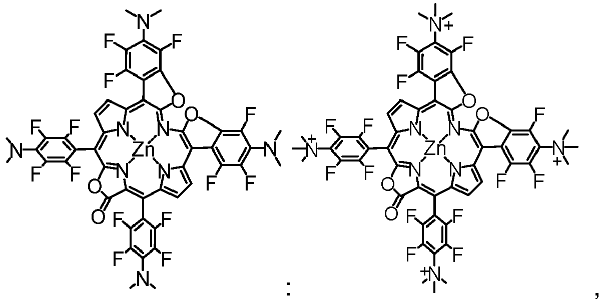

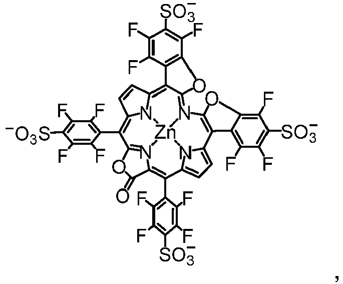

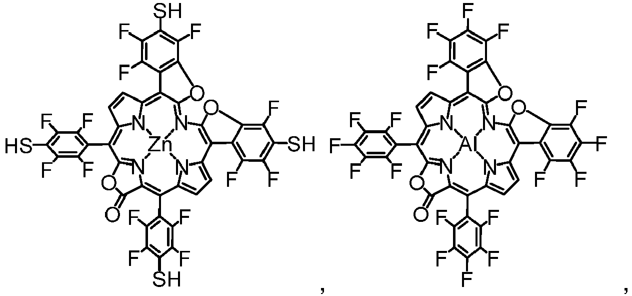

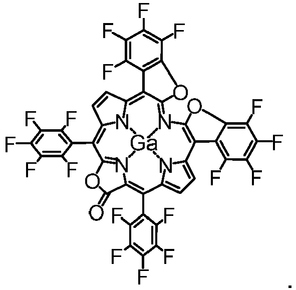

According to the embodiments of the present disclosure, one of the purposes of the present disclosure is to provide a preparation method for the degradable near-infrared photosensitizer described above.

The technical solution of the present disclosure for solving the above technical problem is as follows: the above preparation method for the degradable near-infrared photosensitizer may include:
operation 1: preparing a first intermediate product, including: in a hydrothermal vessel, dissolving 100 mg of porphine dilactone and a base equivalent to 30 - 50 times the porphine dilactone in a range of 5 mL - 20 mL of a first solvent, and reacting at a range of 80 °C - 200 °C for a range of 24 h - 72 h to obtain the first intermediate product, and a reaction equation of the operation 1 may be: 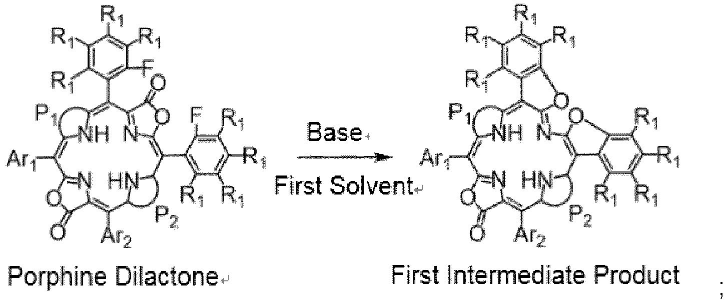
operation 2: preparing a second intermediate product, including: performing a water-soluble modification reaction on 100mg of the first intermediate product obtained in operation 1 in a range of 5 mL-50 mL of a second solvent to obtain the second intermediate product, and a reaction equation of the operation 2 may be: 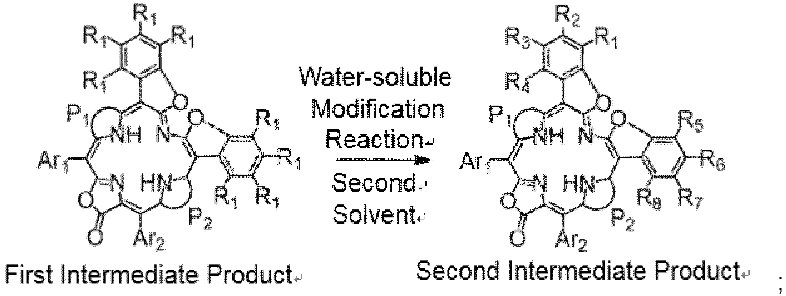
operation 3: preparing a target product, including: dissolving 100 mg of the second intermediate product obtained in operation 2 and a metal salt equivalent to 10 times the second intermediate product in in a range of 20 mL - 100 mL of a third solvent, reacting at a range of 20 °C - 80 °C for a range of 2 h - 16 h to obtain the target product shown in formula 1, and a reaction equation of the operation 3 may be:

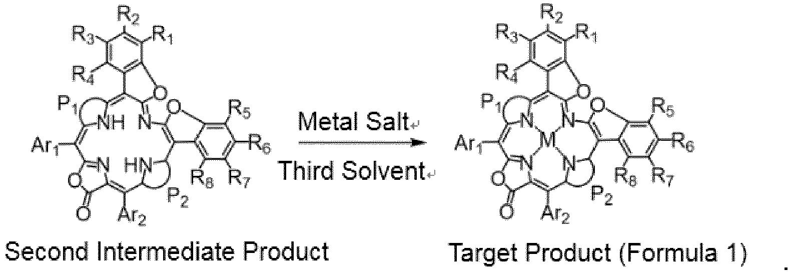

A principle description of the preparation method for the degradable near-infrared photosensitizer of the present disclosure:

In operation 1 of the present disclosure, a porphine dilactone is used as a substrate to convert a lactone group into a ring-conjugated structure under alkaline conditions to increase the degree of conjugation. The fluorine adjacent to the phenyl in the porphine dilactone may undergo a nucleophilic substitution reaction, and under alkaline conditions, the two hydroxyl groups after the ring opening of the lactone moiety can attack the adjacent fluorine to form two new five-membered rings, stabilizing the rigid structure of the macrocycle. The chemical reaction formula involved is:

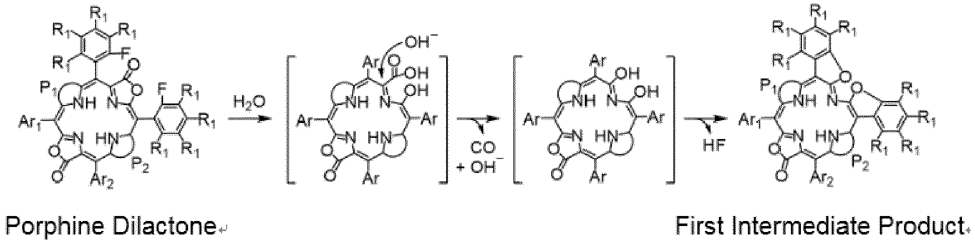

In the operation 2 of the present disclosure, active groups such as amino, hydroxyl, cyano, and sulfhydryl are introduced at R₁ -R₁₃ by a substitution reaction on a benzene ring; and a water-soluble molecule including quaternary ammonium, sulfonic acid, carboxylic acid, sugar groups, and other structures may be obtained by a further modification reaction.

In the operation 3 of the present operation, as tetrapyrrole macrocyclic ligands, the first intermediate product and the second intermediate product may include a good coordination ability and may chelate a variety of metal ions; by selecting different metal ions, target products with different metal centers may be obtained under heating conditions.

The beneficial effect of the preparation method for the degradable near-infrared photosensitizer provided by the present disclosure includes that: (1) an overall synthetic route provided by the present disclosure is simple and has a high yield; and a purification of the photosensitizer by using a recrystallization technology is more simple; (2) the reaction conditions of the present disclosure is mild, the reaction safety is high, such that there is no overflow of harmful substances, thereby reducing pollution; and (3) the preparation method for the degradable near-infrared photosensitizer provided the present disclosure is inexpensive, easy to operate, and has a broad market prospect, which is suitable for large-scale popularization and application.

Based on the above technical solution, the present disclosure may be improved as follows.

In some embodiments, in operation 1, the base may be any one or a mixture of two or more of sodium hydroxide, potassium hydroxide, potassium carbonate, sodium carbonate, triethylamine, N, N-dimethylformamide, sodium methanol, and potassium tert-butoxide.

The further beneficial effects of using the above embodiments include: avoiding a use of a strong base and improving a safety of the reaction.

In some embodiments, the base may be any one or a mixture of two or more of water, methanol, ethanol, acetonitrile, acetone, dimethyl sulfoxide, N, N-dimethylformamide, and tetrahydrofuran.

In some embodiments, in the operation 1, the first solvent may be any one or a mixture of two or more of water, methanol, ethanol, acetonitrile, acetone, dimethyl sulfoxide, N, N-dimethylformamide, and tetrahydrofuran.

In some embodiments, the first solvent may be a mixed solvent of the water and the acetonitrile in a volume ratio of 1 :(2-10), or a mixed solvent of the water and the tetrahydrofuran in a volume ratio of 1 :(2-10).

The beneficial effect of using the above embodiments includes that a reaction yield is increased by using the above parameters.

In some embodiments, the first solvent may be a mixed solvent of the water and the acetonitrile in a volume ratio of 1 :(5-10) or a mixture of the water and the tetrahydrofuran in a volume ratio of 1:(5-10).

In some embodiments, in the operation 1, a temperature of the reaction in operation 1 may be in a range of 100 °C - 150 °C, and a time of the reaction in operation 1 is in a range of 48 h - 72 h.

The beneficial effect of using the above embodiments includes that side reactions are reduced by using the above reaction parameters, which facilitates post-processing and improves a reaction yield.

In some embodiments, in the operation 2, the second solvent may be any one or a mixture of two or more of dimethyl sulfoxide, N, N-dimethylformamide, tetrahydrofuran, ethyl acetate, trimethyl phosphate, triethyl phosphate, and hexanol.

The further beneficial effect of using the above embodiments includes that the second solvent is generated by using the above substances in the operation 2, which facilitates post-processing and improves the reaction yield.

In some embodiments, the second solvent may be a mixture of any one or more of N, N-dimethylformamide, tetrahydrofuran, ethyl acetate, and trimethyl phosphate.

In some embodiments, in the operation 3, the metal salt may be any one of a magnesium salt, a chromium salt, a manganese salt, an iron salt, a cobalt salt, a nickel salt, a copper salt, a zinc salt, a cadmium salt, a gallium salt, an aluminum salt, a palladium salt, and a lead salt.

The further beneficial effect of using the above embodiments includes that a solubility of the above types of metal salts in the third solvent is great and a structure of the target product obtained is stable.

In some embodiments, the metal salt may be any one of the magnesium salt, the zinc salt, the cadmium salt, the gallium salt, and the aluminum salt.

In some embodiments, the metal salt may be any one of acetate, chloride, sulfate, nitrate, and phosphate.

In some embodiments, the metal salt may be any one of acetate, chloride, and nitrate.

In some embodiments, in the operation 3, the third solvent may be any one or a mixture of two or more of water, methanol, ethanol, acetonitrile, acetone, dimethylsulfoxide, N, N-dimethylformamide, tetrahydrofuran, trichloromethane, and methylene chloride.

The beneficial effects of using the above embodiments include: increasing a solubility of the metal salt in the third solvent, reducing a dosage of the third solvent used in the reaction, and simplifying a post-reaction processing.

In some embodiments, the third solvent may be a mixture of any one or more of methanol, ethanol, acetonitrile, and acetone.

In some embodiments, the third solvent may be methanol or ethanol.

In some embodiments, in the operation 3, a temperature of the reaction in the operation 3 may be in a range of 20 °C - 60 °C, and a time of the reaction in the operation 3 may be in a range of 3 h - 5 h.

The further beneficial effect of using the above embodiments includes: shortening the time of the reaction and improving a conversion rate of the reaction.

According to the embodiments of the present disclosure, one of the purposes of the present disclosure is to provide an application of the above degradable near-infrared photosensitizer

The technical solution for solving the above technical problem is as follows: an application of the above degradable near-infrared photosensitizer as an active ingredient in a preparation of the photodynamic therapy medicine.

The beneficial effect of the application of the degradable near-infrared photosensitizer of the present disclosure may be that the degradable near-infrared photosensitizer described above may include a degradable property, which may open up a new mode of photosensitive medicine metabolism, and may realize a new mode of photosensitive medicine metabolism, which is of positive pharmacological significance and social significance.

Based on the above technical solution, the present disclosure may be improved as follows: in some embodiments, the photodynamic therapy medicine may include the above degradable near-infrared photosensitizer and pharmaceutically acceptable excipients, and a dosage form may be any one of powder, injection, and emulsion.

The further beneficial effect of using the above embodiments includes that the photodynamic therapy medicine is made in a variety of dosage forms for a variety of routes of administration.

The degradable near-infrared photosensitizer in the photodynamic therapy medicine may be gathered to a patient site by diffusion, circulation, or targeted medicine delivery, with rapid efficacy, accurate dosage, and reliable action. The route of optimal administration may be an injection administration, and an external administration. The injection may be an intradermal administration, a subcutaneous administration, an intramuscular administration, a local administration, or an intravenous administration; and the external administration may include administration to the patient site by wet coating, brushing, or painting.

In some embodiments, the excipient may be a mixture of any one or more than two of a solvent, an emulsifier, an isotonic modifier, a surfactant, and an antioxidant.

The further beneficial effect of using the above embodiments may be that by using the above excipients, different dosage forms of the medicine may be prepared to improve a stability of the photodynamic therapy medicine, adjust an action of the photodynamic therapy medicine, and improve the biocompatibility. All of the above excipients need to be inactive ingredients with no toxic effect on the human body that are compatible with the route of administration or the mode of administration.

In some embodiments, the solvent may be any one of ethanol, propylene glycol, N, N-dimethylformamide, and dimethyl sulfoxide.

The further beneficial effect of using the above embodiments may include that the solubility of the photodynamic therapy medicine in different dosage forms may be promoted, and in a unit dosage form of the photodynamic therapy medicine, a dosage of the active ingredients is in a range of 0.01 mg to 20 g.

In some embodiments, a surfactant may be any one of polyoxyethylene castor oil, poloxamer, sodium stearate, and tween-80.

The further beneficial effect of using the above embodiments includes that the above surfactant plays roles of emulsification and wetting, which increases the solubility of the active ingredient and the stability of the active ingredient in the dosage form, thereby improving a function of permeation.

### BRIEF DESCRIPTION OF THE DRAWINGS

FIG. 1 is a schematic diagram illustrating a nuclear magnetic resonance hydrogen spectrum of a first intermediate product prepared in Embodiment 1 of the present disclosure;
FIG. 2 is a schematic diagram illustrating a nuclear magnetic resonance hydrogen spectrum of a second intermediate product prepared in Embodiment 2 of the present disclosure;
FIG. 3 is a schematic diagram illustrating a nuclear magnetic resonance hydrogen spectrum of a target product prepared in Embodiment 3 of the present disclosure;
FIG. 4 is a schematic diagram illustrating a nuclear magnetic resonance hydrogen spectrum of a reference compound prepared in Embodiment 4 of the present disclosure;
FIG. 5 is a schematic diagram illustrating a nuclear magnetic resonance hydrogen spectrum of a magnesium complex prepared in Embodiment 5 of the present disclosure;
FIG. 6 is a schematic diagram illustrating a nuclear magnetic resonance hydrogen spectrum of a cadmium complex prepared in Embodiment 5 of the present disclosure;
FIG. 7 is a schematic diagram illustrating a UV-visible absorption spectrum of a target product prepared in Embodiment 3 in Embodiment 6 of the present disclosure;
FIG. 8 is a schematic diagram illustrating a fluorescence emission spectrum of a target product prepared in Embodiment 3 in Embodiment 6 of the present disclosure;
FIG. 9 is a plot diagram illustrating a variation of an emission spectrum of dihydroethidium (DHE) as a function of irradiation time in a presence of a target product prepared in Embodiment 3 in Embodiment 7 of the present disclosure;
FIG. 10 is a plot diagram illustrating a variation of a characteristic emission intensity of dihydroethidium (DHE) as a function of irradiation time in Embodiment 7 of the present disclosure;
FIG. 11 is a plot diagram illustrating a variation of an absorption spectrum of 1,3- diphenylisobenzofuran (DPBF) as a function of irradiation time in a presence of a target product prepared in Embodiment 3 in Experimental Embodiment 7 of the present disclosure;
FIG. 12 is a curve illustrating a degradation rate of a target product prepared in Embodiment 3 versus 1,3-diphenylisobenzofuran (DPBF) in Embodiment 7 of the present disclosure;
FIG. 13 is a plot diagram illustrating a variation of a target product prepared in Embodiment 3 and a variation of 1,3-diphenylisobenzofuran (DPBF) as a function of irradiation time in Embodiment 8 of the present disclosure;
FIG. 14 is a plot diagram illustrating a variation of photodegradation rate of a target product prepared in Embodiment 3 as a function of power intensity in Embodiment 8 of the present disclosure;
FIG. 15 is a schematic diagram illustrating a high-performance liquid chromatogram of a photodegradation process of a target product prepared in Embodiment 3 in Embodiment 8 of the present disclosure;
FIG. 16 is a schematic diagram illustrating a cellular phototoxicity detection of a target product prepared in Embodiment 3 in Embodiment 9 of the present disclosure;
FIG. 17 is a schematic diagram illustrating a cellular phototoxicity detection of a degradation product prepared in Embodiment 3 in Embodiment 9 of the present disclosure;
FIG. 18 is a schematic diagram illustrating a fluorescence signal of a lysosomal probe in Embodiment 10 of the present disclosure;
FIG. 19 is a schematic diagram illustrating a fluorescence signal of a target product prepared in Embodiment 3 in Embodiment 10 of the present disclosure;
FIG. 20 is a schematic diagram illustrating a fluorescence signal after a superimposition of a lysosomal probe and a target product in Embodiment 10 of the present disclosure;
FIG. 21 is a schematic diagram illustrating an in vivo fluorescence imaging of a target product prepared in Embodiment 3 at a depth of 1 mm in Embodiment 11 of the present disclosure;
FIG. 22 is a schematic diagram illustrating an in vivo fluorescence imaging of a target product prepared in Embodiment 3 at a depth of 4 mm in Embodiment 11 of the present disclosure;
FIG. 23 is a schematic diagram illustrating an in vivo fluorescence imaging of a target product prepared in Embodiment 3 at a depth of 7 mm in Embodiment 11 of the present disclosure;
FIG. 24 is a schematic diagram illustrating an in vivo fluorescence imaging of a target product prepared in Embodiment 3 at a depth of 10 mm in Embodiment 11 of the present disclosure;
FIG. 25 is a plot diagram illustrating a variation of a target product prepared in Embodiment 3 and a variation of a tumor volume of a mouse of an irradiation control group in Embodiment 12 of the present disclosure;
FIG. 26 is a plot diagram illustrating a variation of a target product prepared in Embodiment 3 and a variation of a body weight of a mouse of an irradiation control group in Embodiment 12 of the present disclosure;
FIG. 27 is a plot diagram illustrating a variation of a tumor volume of a mouse of a degradation product group prepared in Embodiment 3 in Embodiment 12 of the present disclosure;
FIG. 28 is a plot diagram illustrating a variation of a body weight of a mouse in a degradation product group prepared in Embodiment 3 in Embodiment 12 of the present disclosure;
FIG. 29 is a schematic diagram illustrating a UV-visible absorption spectrum of a target product prepared in Embodiment 3 under continuous irradiation in Comparative Embodiment 1 of the present disclosure;
FIG. 30 is a schematic diagram illustrating a fluorescence emission spectrum of a target product prepared in Embodiment 3 under continuous irradiation in Comparative Embodiment 1 of the present disclosure;
FIG. 31 is a schematic diagram illustrating a UV-visible absorption spectrum of a reference compound prepared in Embodiment 4 in Comparative Embodiment 1 of the present disclosure under continuous irradiation;
FIG. 32 is a schematic diagram illustrating a fluorescence emission spectrum of a reference compound prepared in Embodiment 4 in Comparative Embodiment 1 of the present disclosure under continuous irradiation;
FIG. 33 is a schematic diagram illustrating a cellular imaging of a target product prepared in Embodiment 3 in an irradiation for 0 min in Comparative Embodiment 2 of the present disclosure;
FIG. 34 is a schematic diagram illustrating a cellular imaging of a target product prepared in Embodiment 3 in an irradiation for 5 min in Comparative Embodiment 2 of the present disclosure;
FIG. 35 is a schematic diagram illustrating a cellular imaging of a target product prepared in Embodiment 3 in an irradiation for 10 min in Comparative Embodiment 2 of the present disclosure;
FIG. 36 is a schematic diagram illustrating a cellular imaging of a reference compound prepared in Embodiment 4 in an irradiation for 0 min in Comparative Embodiment 2 of the present disclosure.
FIG. 37 is a schematic diagram illustrating a cellular imaging of a reference compound prepared in Embodiment 4 in an irradiation for 5 min in Comparative Embodiment 2 of the present disclosure;
FIG. 38 is a schematic diagram illustrating a cellular imaging of a reference compound prepared in Embodiment 4 in an irradiation for 10 min in Comparative Embodiment 2 of the present disclosure;
FIG. 39 is a schematic diagram illustrating an in vivo fluorescence imaging of a reference compound prepared in Embodiment 3 in an irradiation for 0 min in Comparative Embodiment 3 of the present disclosure;
FIG. 40 is a schematic diagram illustrating an in vivo fluorescence imaging of a reference compound prepared in Embodiment 3 in an irradiation for 5 min in Comparative Embodiment 3 of the present disclosure;
FIG. 41 is a schematic diagram illustrating an in vivo fluorescence imaging of a reference compound prepared in Embodiment 3 in an irradiation for 10 min in Comparative Embodiment 3 of the present disclosure;
FIG. 42 is a schematic diagram illustrating an in vivo fluorescence imaging of a reference compound prepared in Embodiment 4 in an irradiation for 0 min in Comparative Embodiment 3 of the present disclosure;
FIG. 43 is a schematic diagram illustrating an in vivo fluorescence imaging of a reference compound prepared in Embodiment 4 in an irradiation for 5 min in Comparative Embodiment 3 of the present disclosure;
FIG. 44 is a schematic diagram illustrating an in vivo fluorescence imaging of a reference compound prepared in Embodiment 4 in an irradiation for 10 min in Comparative Embodiment 3 of the present disclosure;
FIG. 45 is a photograph illustrating a paw of a mouse processed by a target product prepared in Embodiment 3 in Comparative Embodiment 4 of the present disclosure;
FIG. 46 is a photograph illustrating a paw of a mouse in an irradiation control group in Comparative Embodiment 4 of the present disclosure;
FIG. 47 is a photograph illustrating a mouse thermal imaging processed by a target product prepared in Embodiment 3 in Comparative Embodiment 4 of the present disclosure;
FIG. 48 is a photograph illustrating a thermal imaging of a mouse in an irradiation control group in Comparative Embodiment 4 of the present disclosure;
FIG. 49 is a photograph illustrating a paw of a mouse processed by a reference compound prepared in Embodiment 4 in Comparative Embodiment 4 of the present disclosure; and
FIG. 50 is a photograph illustrating a mouse thermal imaging processed by a reference compound prepared in Embodiment 4 in Comparative Embodiment 4 of the present disclosure.

### DETAILED DESCRIPTION

The principle and features of the present disclosure will be described below with reference to the accompanying drawings, and the embodiments below are merely used for explaining the present disclosure, and are not intended to limit the scope of the present disclosure;

### Embodiment 1: a preparation of a first intermediate product.

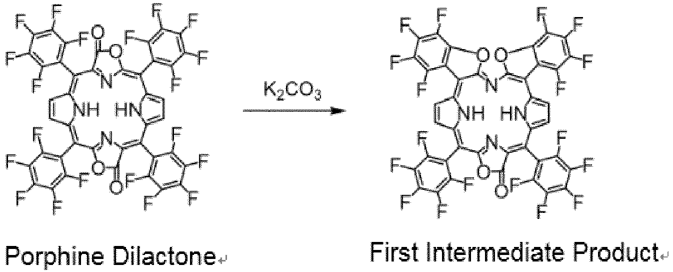

In a vessel, tetrakis (pentafluorophenyl) porphyrin (303 mg, 0.3 mmol) and potassium carbonate (1.24 g, 9 mmol), 7 mL of tetrahydrofuran and 1 mL of water were added to dissolve, and reacted at 80 °C for 24 h to obtain a mixture; heating was stopped, the mixture was cooled naturally to room temperature, a reaction solvent of the mixture was evaporated to obtain a product and the product obtained above was dissolved in dichloromethane, and filtered through diatomaceous earth (200 mesh); the product after filtration was recrystallized by using acetone/petroleum ether; and after filtration and vacuum drying (-0.1Mpa, 10h), 180mg green solid product was obtained, i.e., the first intermediate product.

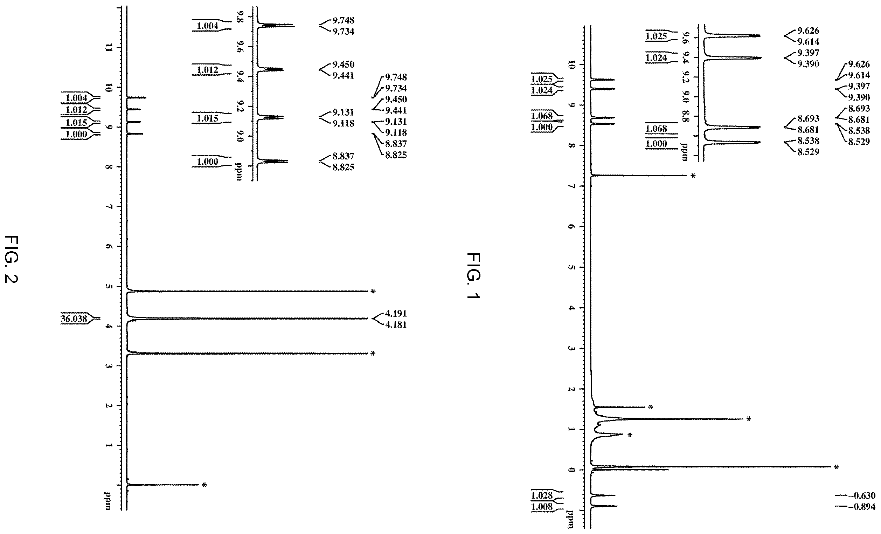

FIG. 1 is a schematic diagram illustrating a nuclear magnetic resonance hydrogen spectrum of a first intermediate product. Characterization data included:
¹H NMR (CDCl₃, 400 MHz): *δ* 9.62 (d, *J* = 4.8 Hz, 1H), 9.39 (d, *J* = 2.8 Hz, 1H), 8.69 (d, *J* = 4.8 Hz, 1H), 8.53 (d, *J* = 3.6 Hz, 1H), -0.63 (s, 1H), -0.89 (s, 1H). ¹⁹F NMR (CDCl₃, 471 MHz): *δ* -58.47 (dd, *J* = 19.7 Hz, *J* = 14.6 Hz, 1F), -59.39 (d, *J* = 6.6 Hz, 1F), -59.44 (d, *J* = 6.1 Hz, 1F), -59.60 (dd, *J* = 19.7 Hz, *J* = 14.6 Hz, 1F), -61.20 (d, *J* = 6.6 Hz, 1F), -61.25 (d, *J* = 7.1 Hz, 1F), -72.48 (t, *J* = 21.2 Hz, 1F), -73.61 (t, *J* = 20.1 Hz, 1F), -75.90 (t, *J* = 20.2 Hz, 1F), -77.55 (t, *J* = 20.2 Hz, 1F), -82.01 (dd, J= 19.7 Hz, *J* = 14.6 Hz, 1F), -82.78 to -82.92 (m, 3F), -83.36 (t, *J* = 20.2 Hz, 1F), -83.66 to -83.82 (m, 3F). HR-MS (ESI⁺) m/z [M+H] ⁺: a determined value was: C₄₁H₇F₁₈N₄O₄ 961.0174; and an experimental value was: 961.0152.

### Embodiment 2: a preparation of a second intermediate product

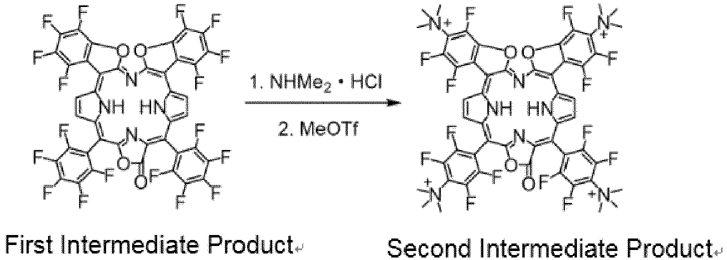

A first intermediate product (94 mg, 0.10 mmol) and dimethylformamide hydrochloride (1.6 g, 20 mmol) were added to 5 mL of N, N-dimethylformamide to obtain a mixture, a reflux reaction was performed on the mixture for 36 h, after the reaction, the mixture was cooled to room temperature, 50 mL of deionized water was added to the mixture for precipitation, filtration and vacuum drying (-0.1 Mpa, 10 h), and a product was obtained; under a protection of nitrogen, 80 mg of the product obtained after the above vacuum drying and 0.5 mL of methyl trifluoromethanesulfonate were added to 5 mL of trimethyl phosphate to obtain a mixture, the mixture reacted at the room temperature for 24 h, 50 mL of methylene chloride was added to the mixture for precipitation and centrifugation (4000r, 10min) to obtain a solid, the solid was rinsed by using 20 mL of methylene chloride and dried under vacuum (-0.1Mpa, 10h), and 100 mg of a purple solid product, i.e., the second intermediate product was obtained.

FIG. 2 is a schematic diagram illustrating a nuclear magnetic resonance hydrogen spectrum of a second intermediate product. Characterization data included:
¹H NMR (CD₃OD, 400 MHz): *δ* 9.74 (d, *J* = 5.6 Hz, 1H), 9.45 (d, *J* = 3.6 Hz, 1H), 9.13 (d, *J* = 5.2 Hz, 1H), 8.83 (d, *J* = 4.8 Hz, 1H), 4.19 (d, *J* = 4.0 Hz, 36H). ¹⁹F NMR (CD₃OD, 471 MHz): *δ* -135.25 (t, *J* = 15.0 Hz, 1F), -136.12 (dd, *J* = 26.3 Hz, *J* = 12.2 Hz, 2F), -136.88 (t, *J* = 16.0 Hz, 1F), -137.56 (d, *J* = 11.8 Hz, 1F), -137.62 (d, *J* = 13.1 Hz, 1F), -138.01 (t, *J* = 14.1 Hz, 1F), -138.31 (d, *J* = 15.5 Hz, 2F), -139.32 (d, *J* = 15.0 Hz, 2F), -140.62 (t, *J* = 14.6 Hz, 1F), -141.49 (t, *J=* 13.2 Hz, 1F). HR-MS (ESI⁺) m/z [M]⁴⁺: a determined value was: C₅₃H₄₂F₁₄N₈O₄⁴⁺ 280.0771 ; and an experimental value was: 280.0767.

### Embodiment 3: a preparation of a target product

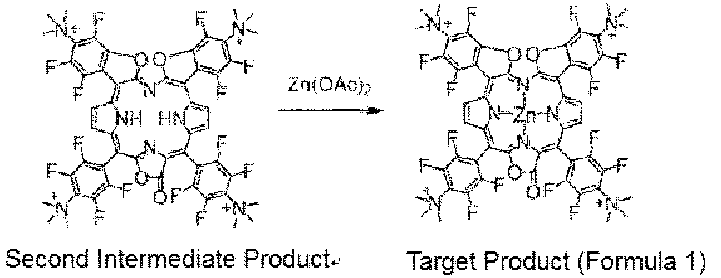

The second intermediate product (110 mg, 0.1 mmol) and zinc acetate (200 mg, 1 mmol) were dissolved in 20 mL of methanol to obtain a mixture. The mixture was stirred at room temperature overnight, and then the solvent of the mixture was evaporated to obtain a product. The product obtained by evaporating the solvent of the mixture was dissolved in 5 mL of acetonitrile for centrifugation (4000r, 5min), a supernatant was collected, 50 mL of methylene chloride was added to the mixture for precipitation, centrifuged (4000r, 10min), and vacuum drying (-0.1 Mpa, 10h), and 110 mg of a green solid product was obtained, which is the target product, i.e., molecules of a degradable near-infrared photosensitizer.

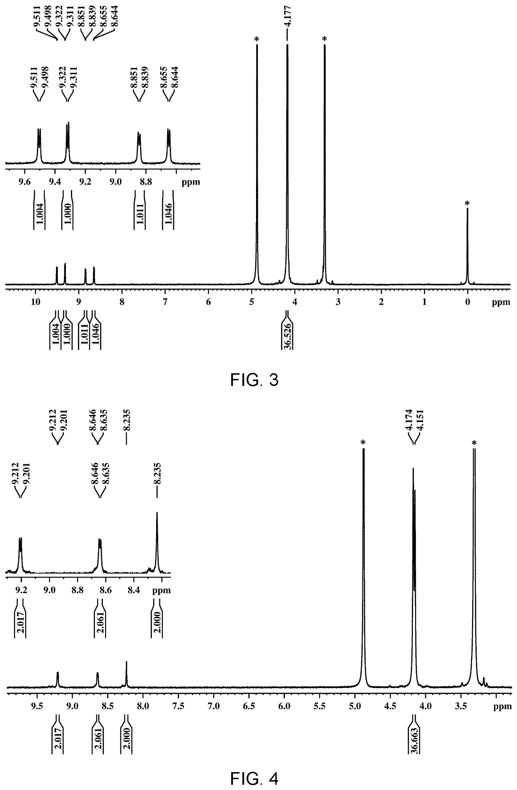

FIG. 3 is a schematic diagram illustrating a nuclear magnetic resonance hydrogen spectrum of the target product. Characterization data included:
¹H NMR (CD₃OD, 400 MHz): *δ* 9.51 (d, *J* = 5.2 Hz, 1H), 9.32 (d, *J* = 4.4 Hz, 1H), 8.85 (d, *J* = 4.8 Hz, 1H), 8.65 (d, *J* = 4.4 Hz, 1H), 4.18 (s, 36H). ¹⁹F NMR (CDsOD, 471 MHz): *δ* -136.39 (dd, *J* = 27.7 Hz, *J* = 12.7 Hz, 2F), -136.71 (t, *J* = 14.6 Hz, 1F), -137.13 (t, *J* = 14.6 Hz, 1F), -137.95 (dd, *J* = 28.2 Hz, *J* = 13.2 Hz, 2F), -138.78 (d, *J* = 15.5 Hz, 2F), -138.90 (s, 1F), -138.99 (s, 1F), -140.03 (d, *J* = 16.0 Hz, 2F), -141.28 (t, *J* = 13.2 Hz, 1F), -142.04 (t, *J* = 12.7 Hz, 1F). HR-MS (ESI⁺) m/z [M]⁴⁺: a determined value was: C₅₃H₄₀F₁₄N₈O₄Zn⁴⁺ 295.5555; and an experimental value was: 295.5557.

### Embodiment 4: a preparation of a reference compound.

The reference compound, which is a non-degradable photosensitizer, was prepared using tetrakis (pentafluorophenyl) porphyrin (298 mg, 0.3 mmol) as a starting reactant, and other operations in embodiment 4 were the same as the operations in Embodiments 1-3.

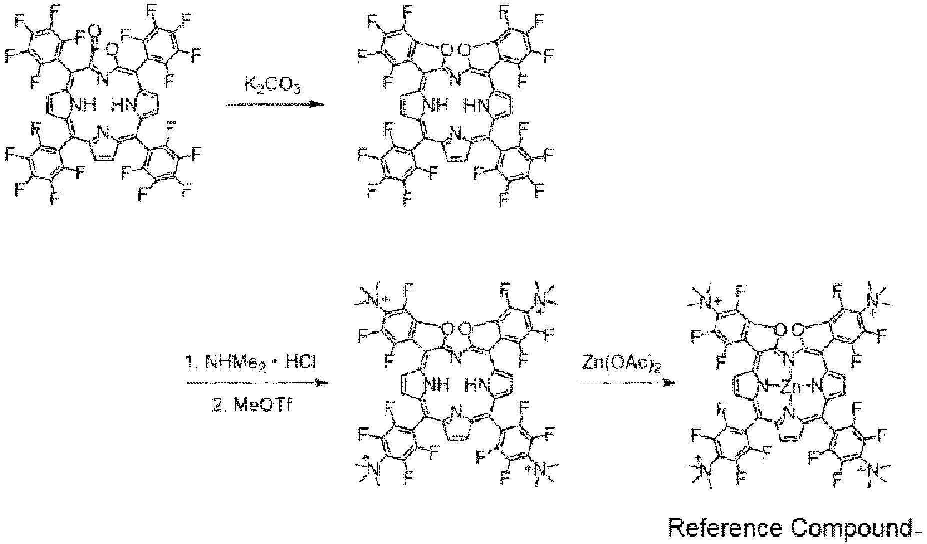

FIG. 4 is a schematic diagram illustrating a nuclear magnetic resonance hydrogen spectrum of the reference compound. Characterization data included:
¹H NMR (CD₃OD, 400 MHz): *δ* 9.21 (d, *J* = 4.4 Hz, 2H), 8.64 (d, *J* = 4.4 Hz, 2H), 8.24 (s, 2H), 4.16 (d, *J* = 9.2 Hz, 36H). ¹⁹F NMR (CDsOD, 471 MHz): *δ* -137.15 (dd, *J* = 34.8 Hz, *J* = 16.9 Hz, 4F), -138.82 (t, *J* = 20.7 Hz, 2F), -139.49 (d, *J* = 20.2 Hz, 4F), -139.64 (s, 2F), -142.36 (t, *J* = 14.6 Hz, 2F). HR-MS (ESI⁺) m/z [M]⁴⁺: a determined value was: C₅₄H₄₂F₁₄N₈O₂Zn⁴⁺ 291.0619; and an experimental value was: 291.0620.

### Embodiment 5: a preparation of different metal-centered complexes

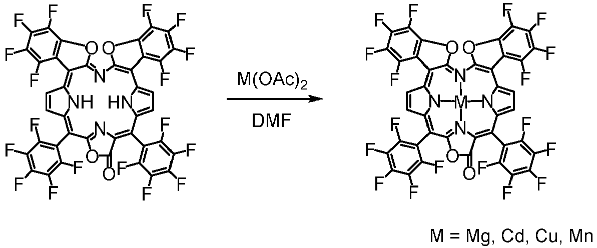

The first intermediate product (110 mg, 0.1 mmol) and magnesium acetate/cadmium acetate/copper acetate/manganese acetate (10 eq.) were dissolved in 10 mL of N, N-dimethylformamide. The mixture reacted at 150 °C for 8 h; the heating was stopped, the mixture was cooled naturally to room temperature, a reaction solvent of the mixture was evaporated to obtain a product, and the product obtained above was dissolved in dichloromethane, and filtered through diatomaceous earth (200 mesh); the product after filtration was recrystallized acetone/petroleum ether to obtain complexes with the central metals of magnesium, cadmium, copper, and manganese were obtained, respectively.

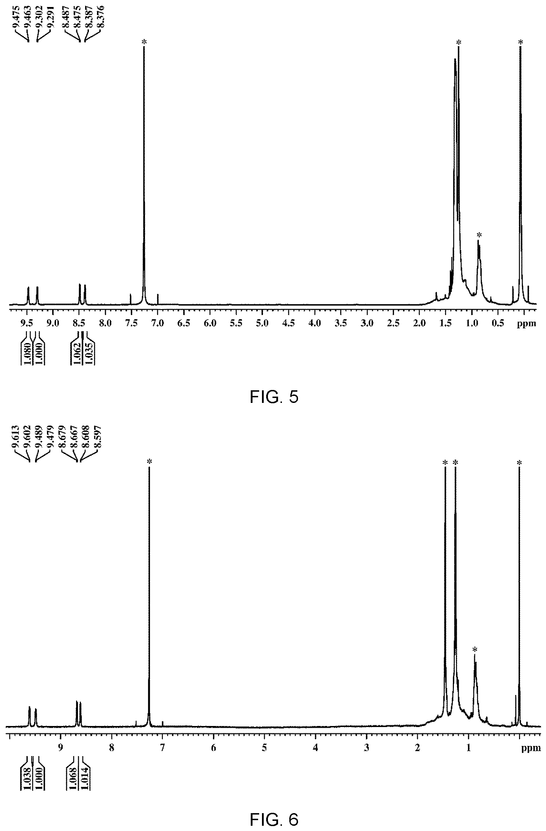

FIG. 5 is a schematic diagram illustrating a nuclear magnetic resonance hydrogen spectrum of a magnesium complex. Characterization data included:
¹H NMR (CDCl₃, 400 MHz): *δ* 9.47 (d, *J* = 4.8 Hz, 1H), 9.29 (d, *J* = 4.4 Hz, 1H), 8.48 (d, *J* = 4.8 Hz, 1H), 8.38 (d, *J* = 4.4 Hz, 1H). HR-MS (ESI⁺) m/z [M+H] ⁺: a determined value was: C₄₁H₅F₁₈MgN₄O₄ 982.9868; and an experimental value was: 982.9887.

FIG. 6 is a schematic diagram illustrating a nuclear magnetic resonance hydrogen spectrum of a cadmium complex. Characterization data included:
¹H NMR (CDCl₃, 400 MHz): *δ* 9.61 (d, *J* = 4.4 Hz, 1H), 9.48 (d, *J* = 4.0 Hz, 1H), 8.67 (d, *J* = 4.8 Hz, 1H), 8.60 (d, *J* = 4.4 Hz, 1H). HR-MS (ESI⁺) m/z [M+H] ⁺: a determined value was: C₄₁H₅CdF₁₈N₄O₄ 1072.9063; and an experimental value was: 1072.9077.

The Copper complex: HR-MS (ESI⁺) m/z [M+H] ⁺: a determined value was: C₄₁H₅CuF₁₈N₄O₄ 1021.9314; and an experimental value was:1021.9299.

The Manganese complex: HR-MS (ESI⁺) m/z [M+H] ⁺: a determined value was: C₄₁H₅F₁₈MnN₄O₄ 1013.9398; and an experimental value was:1013.9410.

### Embodiment 6: a detection of photophysical property

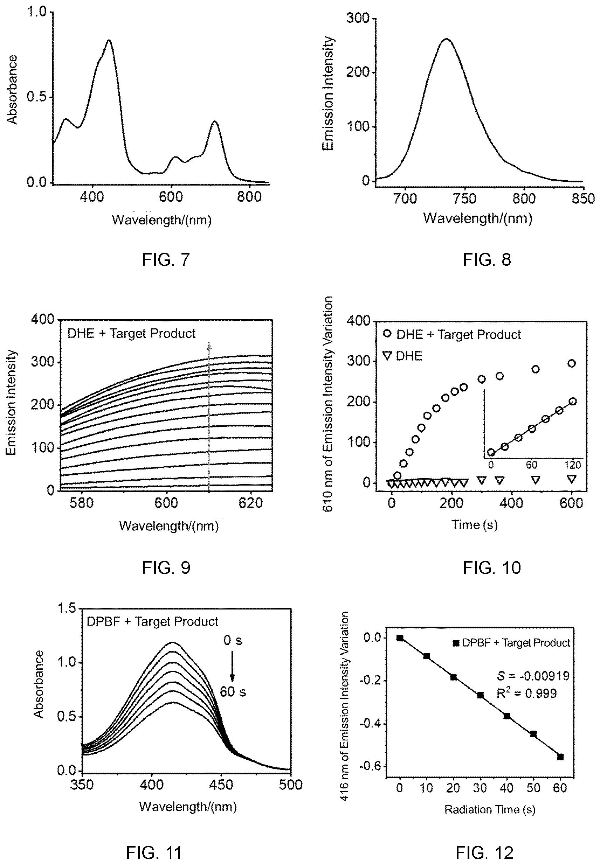

A UV-visible absorption spectrum scanning and an emission spectrum scanning were performed on the target product prepared in Embodiment 3, and the scanning results are shown in FIGs. 7 and 8. As shown in FIG. 7, an absorption spectrum of the target product covered a visible region and a near-infrared region, and the absorption spectrum of the target product included a strong absorption in a red-near-infrared region, with a range of 600 nm - 750 nm. As shown in FIG. 8, a fluorescence spectrum of the target product was also in the near-infrared region when a light excitation was performed on the target product. Therefore, the target product may include a deep tissue penetration depth in photodynamic therapy or in vivo fluorescence imaging.

### Embodiment 7: a detection of reactive oxygen species

Under an air atmosphere, dihydroethidium (DHE) was added to a water solution (10 µM) of the target product prepared in Embodiment 3 as a probe of superoxide anion free radicals, and an irradiation condition was a 700 nm light-emitting diode lamp of 1.25 mW/cm², a fluorescence of ethidium oxide generated by an oxidation of the DHE by the superoxide radicals was detected, and a fluorescence intensity enhanced with the increase of an irradiation time. The results are shown in FIGs. 9 and 10, indicating that the target product may include an ability to generate the superoxide anion free radicals under irradiation.

A phosphorescence emission of singlet oxygen was easily quenched by water molecules, and the singlet oxygen generated by the target product was detected by using chemical capture spectrophotometry. 1,3-diphenylisobenzofuran (DPBF) was one of the most commonly used singlet oxygen capture agents, and 1,3-diphenylisobenzofuran reacted with the singlet oxygen rapidly to generate a colorless substance, and a content of the singlet oxygen may be reflected by detecting a variation of an absorbance of the 1,3-diphenylisobenzofuran (DPBF) at a characteristic absorption peak (416 nm) using the UV-visible absorption spectrum. An irradiation condition was a 700 nm light-emitting diode (LED) lamp of 1.25 mW/cm², a characteristic absorption of the 1,3-diphenylisobenzofuran (DPBF) at the characteristic absorption peak of 416 nm was gradually weakened. The results are shown in FIGs. 11 and 12, indicating that the target produced the ability to produce singlet oxygen under irradiation.

### Experiment example 8: a monitoring of self-degradation

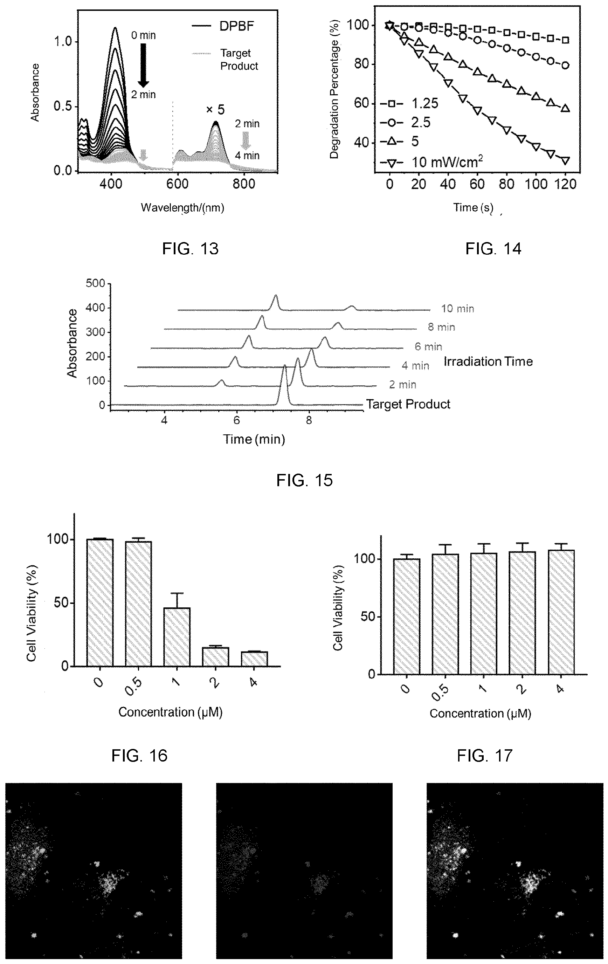

In the test of Embodiment 2, an irradiation condition was the 700 nm light-emitting diode lamp of 1.25 mW/cm² for 0 min - 2 min, the characteristic absorption of 1,3-diphenylisobenzofuran (DPBF) at the characteristic absorption peak of 416 nm was gradually weakened. An irradiation intensity was increased and the irradiation time was prolonged, the irradiation condition was a 700 nm light-emitting diode lamp of 10 mW/cm² for 2 min - 4 min, the characteristic absorption of the target product at characteristic absorption peaks of 440 nm and 710 nm decreased significantly. The results are shown in FIG. 13, indicating that the target product degraded under irradiation. In addition, as shown in FIG. 14, the degradation rate was accelerated with the increase in irradiation intensity.

A photodegradation process was further monitored by HPLC, with a prolongation of irradiation time, the irradiation condition was 700 nm light-emitting diode lamp of 2.5 mW/cm² for 0 min - 10 min, the characteristic peaks of the target product gradually decreased, and a new characteristic peak (a degradation product) was generated at 5 min and gradually enhanced, the degradation product was collected. The results are shown in FIG. 15, indicating that the target product was able to self-degrade and gradually be converted into the degradation product.

### Embodiment 9: a detection of cellular phototoxicity

The cells used in the experiment were HeLa human cervical cancer cells. Cell culture was performed in a DMEM complete culture medium (purchased from Genye, 500 mL) supplemented with 10% inactivated fetal bovine serum (purchased from Shuohua, 500 mL) with 1% penicillin-streptomycin (purchased from Corning, 100 mL) at a temperature of 37 °C and an atmosphere of 5% carbon dioxide.

Sub-cultured HeLa cells were digested by trypsin and dispersed in the culture medium at an appropriate concentration. Dispersed HeLa cells were inoculated into flat-bottomed 96-well plates such that the culture medium was 200 µL per well, a count of cells per well was about 10⁴, and a set of cell-free culture medium was kept as blank control. After culturing the cells in a dark environment for 24 h, the culture medium was removed, a solution of 100 µL of fresh culture medium mixed with 100 µL of pre-prepared target product was added, or a solution of 100 µL of fresh culture medium mixed with 100 µL of degradation product prepared in Embodiment 3 was added, so that a sample was a gradient concentration in a range of 0 µM - 4 µM after dilution. After culturing the cells in the dark environment for 24 h, the culture medium was removed, and each well was rinsed three times using PBS buffer at pH 7.4.

100 µL of PBS buffer was added to each well, and an irradiation condition was a 700 nm light-emitting diode lamp of 2.5 mW/cm² for 10 min. The PBS buffer was removed from each well and replaced with 200 µL of fresh culture medium, and the cells were continued to culture for 24 h. After a completion of 24 h of the culture, the culture medium was removed and the wells were lubricated with PBS buffer 3 times. Then 10% of a Cell Counting Kit-8 reagent (CCK-8 reagent) was configured with the culture medium and 100 µL of the CCK-8 reagent was added to each well and cultured for 2 h. During this process, a monosodium salt of 2-(2-methoxy-4-nitrophenyl)-3-(4-nitrophenyl)-5-(2,4-disulfonate phenyl)-2H-tetrazolium (WST-8) in the CCK-8 reagent with an electron coupling reagent may be reduced to a water-soluble yellow formazan product by living cells, which causes an absorbance of the solution at 450 nm to vary, and values of variation may be proportional to a count of living cells. The variation of the absorbance at 450 nm of each well was measured using an enzyme labeling apparatus, and a cell viability at each incubation concentration was calculated according to a formula:
CV=(As-Ab)/(Ac-Ab)×100%. A CV may refer to the cell viability. As, Ac and Ab may refer to an absorbance of the cells of an incubated compound, an absorbance of blank cells, and an absorbance of the blank control, respectively.

A half-lethal concentration IC₅₀ of the target product in HeLa cells was determined as 1.0 µM from the cell viability at each incubation concentration. FIG. 16 is a schematic diagram illustrating a cellular phototoxicity detection of the target product. As shown in FIG. 16, the target product may include a relatively strong cellular phototoxicity under irradiation. FIG. 17 is a schematic diagram illustrating a cellular phototoxicity detection of a degradation product. As shown in FIG. 17, the degradation product may not include the phototoxicity.

### Embodiment 10: a detection of cell fluorescence imaging

The sub-cultured HeLa cells were digested by trypsin and dispersed in the culture medium at the appropriate concentration. The dispersed HeLa cells were inoculated into a petri dish, and after incubating the cells for 24 h, 100 µL of pre-prepared culture medium solution of the target product was added and diluted so that the sample was at a concentration of 10 µM. After 12 h of incubation, a lysosomal green fluorescence probe was added, the culture medium was removed after 15 min of co-incubation, and the sample was lubricated three times using PBS buffer with pH 7.4. Fluorescence imaging was performed with an ISS-integrated laser scanning confocal fluorescence lifetime imaging system with a supercontinuum laser excitation at 700 nm and a 715 nm high-pass filtration to collect the fluorescence signals of the target product, and a laser excitation at 488 nm and a 525/50 nm band-pass filtration to collect the fluorescence signals of the lysosomal probe.

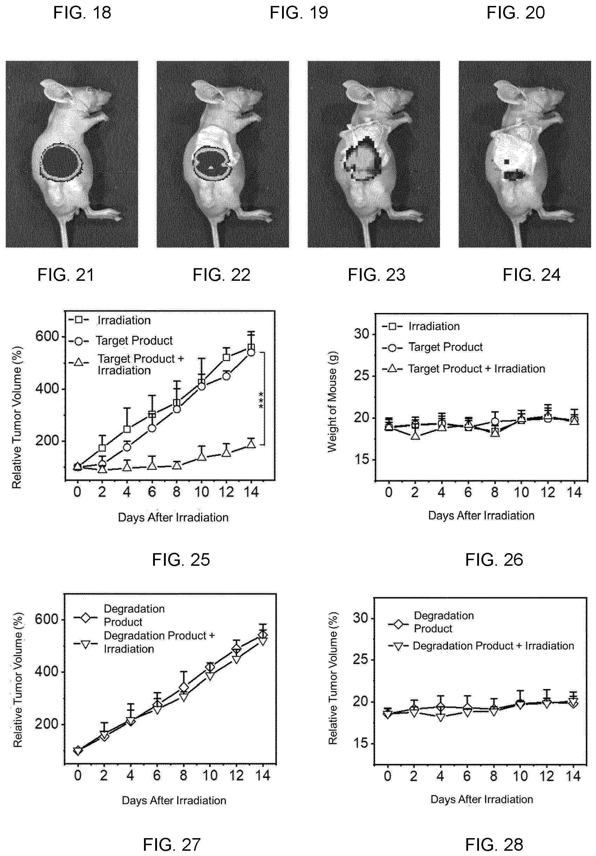

FIG. 18 is a schematic diagram illustrating a fluorescence signal of a lysosomal probe. As shown in FIG. 18, the lysosomal probe may display a relatively strong fluorescence signal in a green light channel.

FIG. 19 is a schematic diagram illustrating a fluorescence signal of a target product. As shown in FIG. 19, the target product may include a relatively strong fluorescence signal of the target product in the near-infrared region.

FIG. 20 is a schematic diagram illustrating a fluorescence signal after a superimposition of a lysosomal probe and a target product. As shown in FIG. 20, a near-infrared fluorescence of the target product and a green fluorescence of the lysosomal probe may overlap well, which indicates that the target product may include a potential to be used as a probe of near-infrared living cell fluorescence imaging and may be in the lysosome.

### Embodiment 11: a detection of depth of in vivo fluorescence imaging

All animal experiments in the in vivo experiments were performed in strict compliance with the Chinese regulations for animal experiments using BALB/C nude mice, 5 weeks of age of the mouse, male, and a range of 16 g - 25 g of body weight. 100 µL (0.2 mg/mL) of PBS solution of the target product was injected subcutaneously into an abdominal region of the mouse, and a dose of 0.2 mg/kg was administered. After 30 min, the mouse was placed in an imaging apparatus under a mixed gas atmosphere of 2 L /min of oxygen and 2% isoflurane gas mixture atmosphere to anesthetize the mouse. A target product excitation wavelength was 710 nm, and an image collecting wavelength was 750 nm. The mouse skin (1 mm/layer) was superimposed on the injection site, and variations in the intensity of a fluorescence signal were monitored until the signal disappeared.

FIG. 21 is a schematic diagram illustrating an in vivo fluorescence imaging of a target product at a depth of 1 mm. As shown in FIG. 21, the target product exhibited a strong fluorescence signal under the skin of the mouse.

FIG. 22 is a schematic diagram illustrating an in vivo fluorescence imaging of a target product at a depth of 4 mm. As shown in FIG. 22, a detectable fluorescence signal of the target product was slightly attenuated.

FIG. 23 is a schematic diagram illustrating an in vivo fluorescence imaging of a target product at a depth of 7 mm. As shown in FIG. 23, a detectable fluorescence signal of the target product was further weakened with an increase in imaging depth.

FIG. 24 is a schematic diagram illustrating an in vivo fluorescence imaging of a target product at a depth of 10 mm. As shown in FIG. 24, a fluorescence signal of the target product may be still effectively detected at a depth of 10 mm.

### Embodiment 12: a medicine phototherapy at an animal level

All animal experiments in the in vivo experiments were performed in strict compliance with the Chinese regulations for animal experiments using BALB/C nude mice, 5 weeks of age of the mouse, male, and a range of 16 g - 25 g of body weight. Each mouse was subcutaneously inoculated with 100 µL of HeLa human cervical cancer cells in the right hind limb (5×10⁶) to construct the tumor model, and the experiments were performed two weeks later.

Mice were randomly grouped by number, and the experiment was divided into an irradiation control group (including irradiation), a dark control group (including a target product; and a degradation product), and an experimental group (including target product + irradiation; and degradation product + irradiation), with six mice in each group. The dark control and experimental groups were administered by in situ injection, respectively: a dose of 0.2 mg/kg was administered, and 20 µL (0.2 mg/mL) of PBS solution of the target product or degradation product was injected in situ. After 30 min, the irradiation control and experimental groups were irradiated, and an irradiation condition was a 700 nm light-emitting diode lamp of 100 mW/cm², and 10 min of irradiation per mouse. The six mice were not given a light-avoidance process after therapy and were put into rearing cages to continue to feed, and every two days, a tumor volume was measured by vernier calipers and the body weight was weighed. The mice were therapized for 2 weeks.

FIG. 25 is a plot diagram illustrating a variation of a target product and a variation of a tumor volume of a mouse of an irradiation control group. As shown in FIG. 25, the target product effectively inhibited a growth of a subcutaneous tumor under a single irradiation condition, and a mouse tumor in an irradiation control group to which the target product was not administered and a dark control group to which the target product was administered grows rapidly.

FIG. 26 is a plot diagram illustrating a variation of a target product and a variation of a body weight of a mouse of an irradiation control group. As shown in FIG. 26, there was no significant weight loss of mice in either the experimental groups or control groups.

The variation in tumor volume of mice in the degradation product group was plotted as shown in FIG. 27. The degradation products did not inhibit tumor growth under irradiation as well as dark control conditions, indicating that the degradation products were not photoactive and did not cause damage to cells and tissues.

FIG. 28 is a plot diagram illustrating a variation of a body weight of a mouse in a degradation product group. As shown in FIG. 28, there was no significant weight loss in the irradiation control group of the degradation product and the dark control group of the degradation product, indicating that the degradation product had no significant side effects on the organism.

### Comparative Embodiment 1: a photodegradation process of a photosensitizer in solution

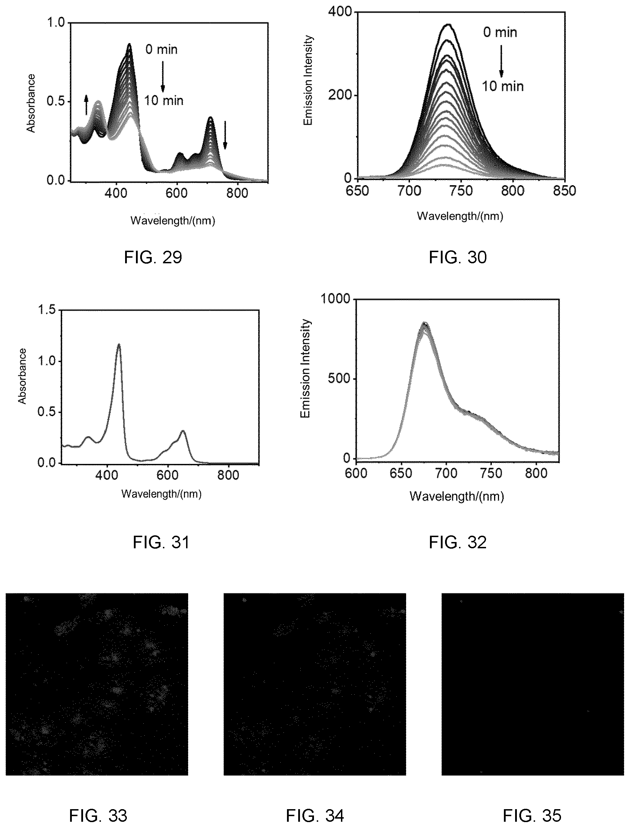

The target product obtained in Embodiment 3 was placed under a light-emitting diode lamp for irradiation, and an irradiation condition was 700 nm of 10 mW/cm², and the UV-visible absorption spectrum and an emission spectrum of the target product were recorded during irradiation. As shown in FIG. 29, the characteristic absorption of the target product at 440 nm and 710 nm was reduced. As shown in FIG. 30, a characteristic emission of the target product at 740 nm was also gradually weakened. The results indicated that the target product was degraded under irradiation.

As a control, the reference compound prepared in Embodiment 4 was placed under a light-emitting diode lamp for irradiation, the irradiation condition was 640 nm, 10 mW/cm², and the UV-visible absorption spectrum and the emission spectrum of the reference compound were recorded during irradiation.

FIG. 31 is a schematic diagram illustrating a UV-visible absorption spectrum of a reference compound under continuous irradiation. As shown in FIG. 31, the absorption spectrum of the reference compound did not vary under continuous irradiation conditions.

FIG. 32 is a schematic diagram illustrating a fluorescence emission spectrum of a reference compound. As shown in FIG. 32, there was no significant variation in the fluorescence emission intensity of the reference compound, which indicated that the reference compound does not degrade under an irradiation condition.

### Comparative Embodiment 2: a photodegradation process of a photosensitizer in cells

The HeLa cells were inoculated into a petri dish, and after incubating the cells for 24 h, 100 µL of pre-prepared culture medium solution of the target product or the reference compound was added to the petri dish, and the sample was diluted at a concentration of 10 µM. After 12h of incubation, the cells were rinsed three times using PBS buffer at pH 7.4. The fluorescence imaging was performed with an ISS-integrated laser scanning confocal fluorescence lifetime imaging system with an excitation at 405 nm and a 715 nm high-pass filtration to collect the fluorescence signals of the target product; and with an excitation at 405 nm and a 670/50 nm bandpass filtration to collect the fluorescence signals of the reference compound.

FIG. 33 is a schematic diagram illustrating a cellular imaging of a target product in an irradiation for 0 min. As shown in FIG. 33, the target product included a relatively strong fluorescence signal of the target product in a near-infrared region. The petri dish was irradiated under a light-emitting diode lamp with irradiation conditions of 700 nm, 10 mW/cm², and imaging was performed every 5 min.

FIG. 34 is a schematic diagram illustrating a cellular imaging of a target product in an irradiation for 5 min. As shown in FIG. 34, after an irradiation for 5 min, the fluorescence signal of the target product was significantly weakened.

FIG. 35 is a schematic diagram illustrating a cellular imaging of a target product in an irradiation for 10 min. As shown in FIG. 35, the fluorescence signals of the target product were completely quenched after an irradiation for 10 min, indicating that the target product in the cell underwent self-degradation under irradiation.

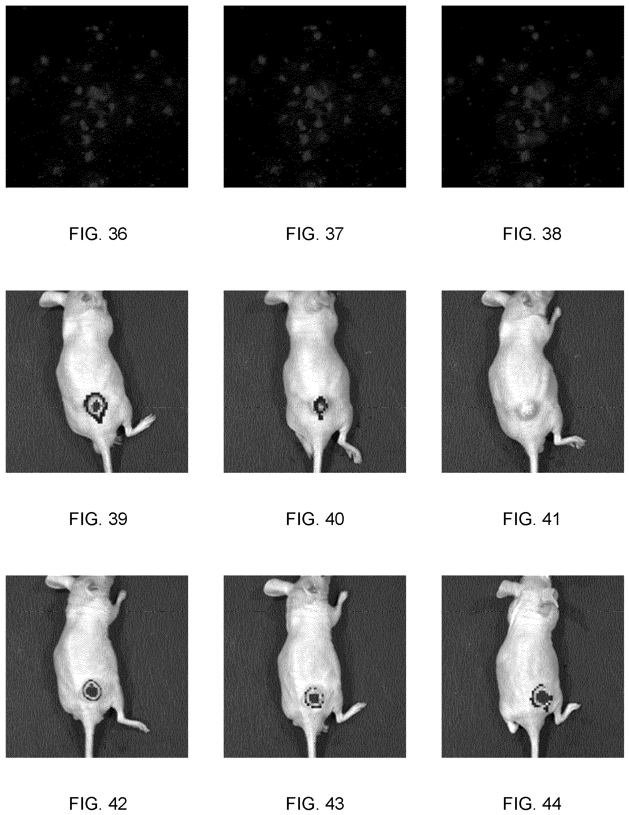

As a control, FIG. 36 is a schematic diagram illustrating a cellular imaging of a reference compound in an irradiation for 0 min. As shown in FIG. 36, the reference compound exhibited a relatively strong fluorescence signal. The petri dish was placed under a light-emitting diode lamp irradiation with an irradiation condition of 640 nm, 10 mW/cm², and an imaging was performed every 5 min.

FIG. 37 is a schematic diagram illustrating a cellular imaging of a reference compound in an irradiation for 5 min. As shown in FIG. 37, after an irradiation for 5 min, an intensity of a fluorescence signal of the reference compound did not diminish.

FIG. 38 is a schematic diagram illustrating a cellular imaging of a reference compound in an irradiation for 10 min. As shown in FIG. 38, there was no significant variation in the intensity of the fluorescence signal of the reference compound after an irradiation for 10 min.

### Comparative Embodiment 3: a photodegradation process of a photosensitizer at an animal level

All animal experiments in the in vivo experiments were performed in strict compliance with the Chinese regulations for animal experiments, using BALB/C nude mice, 5 weeks of age of the mouse, male, and a range of 16 g - 25 g of body weight. Each mouse was subcutaneously inoculated with 100 µL of HeLa human cervical cancer cells in a right hind limb (5×10⁶) to construct the tumor model, and the experiments were performed two weeks later.

A PBS solution of 20 µL (0.2 mg/mL) of the target product or reference compound was injected in situ into a mouse tumor, and the administered dose was 0.2 mg/kg. After 30 min, a mouse was anesthetized by placing the mouse in an imaging apparatus at a mixed gas atmosphere of 2 L/min of oxygen and 2% isoflurane. A target product excitation wavelength was 710 nm, and a wavelength of an image collecting wavelength was 750 nm. A reference compound excitation wavelength was 650 nm, and an image collecting wavelength was 690 nm.

FIG. 39 is a schematic diagram illustrating an in vivo fluorescence imaging of a reference compound in an irradiation for 0 min. As shown in FIG. 39, the target product exhibited a relatively strong fluorescence signal within the mouse tumor. A mouse tumor site was irradiated under a light-emitting diode lamp with an irradiation condition of 700 nm, 100 mW/cm², and an imaging was performed every 5 min.

FIG. 40 is a schematic diagram illustrating an in vivo fluorescence imaging of a reference compound in an irradiation for 5 min. As shown in FIG. 40, the fluorescence signal inside the mouse tumor was significantly weakened after an irradiation for 5 min, indicating that the target product underwent a self-degradation while undergoing irradiation therapy.

FIG. 41 is a schematic diagram illustrating an in vivo fluorescence imaging of a reference compound in an irradiation for 10 min. As shown in FIG. 41, the fluorescence signal in the mouse tumor was completely quenched after an irradiation for 10 min, and when the therapy was completed, the fluorescence signal in the mouse tumor was completely converted to a degradation product without photoactivity.

FIG. 42 is a schematic diagram illustrating an in vivo fluorescence imaging of a reference compound in an irradiation for 0 min. As shown in FIG. 42, the reference compound exhibited a relatively strong fluorescence signal inside the mouse tumor. The mouse tumor site was irradiated under a light-emitting diode lamp with an irradiation condition of 640 nm, 100 mW/cm², and an imaging was performed every 5 min.

FIG. 43 is a schematic diagram illustrating an in vivo fluorescence imaging of a reference compound in an irradiation for 5 min. As shown in FIG. 43, the fluorescence signal inside the mouse tumor did not diminish after an irradiation for 5 min.

FIG. 44 is a schematic diagram illustrating an in vivo fluorescence imaging of a reference compound in an irradiation for 10 min. As shown in FIG. 44, there was no significant variation in an intensity of a fluorescence signal in the mouse tumor after an irradiation for 10 min, indicating that the reference compound did not include photodegradation properties.

### Comparative Embodiment 4: photosensitization side effects of a photosensitizer at an animal level.

All animal experiments in the in vivo experiments were performed in strict compliance with the Chinese regulations for animal experiments, using BALB/C nude mice, 5 weeks of age of the mouse, male, and a range of 16 g - 25 g of body weight.

The mice were randomly grouped by number, and the experiment was divided into a target product group, a reference compound group, and an irradiation control group, with 3 mice in each group. Each of the mice was injected subcutaneously into an abdomen with 20 µL (0.2 mg/mL) of the target product, reference compound solution, or PBS solution, and an administered dose was 0.2 mg/kg. After the injection, a site of administration was irradiated, an irradiation condition of the target product was a 700 nm light-emitting diode lamp, and an irradiation condition of the reference compound was a 640 nm light-emitting diode lamp of 100 mW/cm², each of the mice was irradiated for 10 min, and no processing was performed in the irradiation control group. The state of the mouse was observed and a body temperature of the mouse was recorded with a thermal imager. Further, the three groups of mice were exposed to a simulated natural light of 10mW/cm² and irradiated for 1 hour.

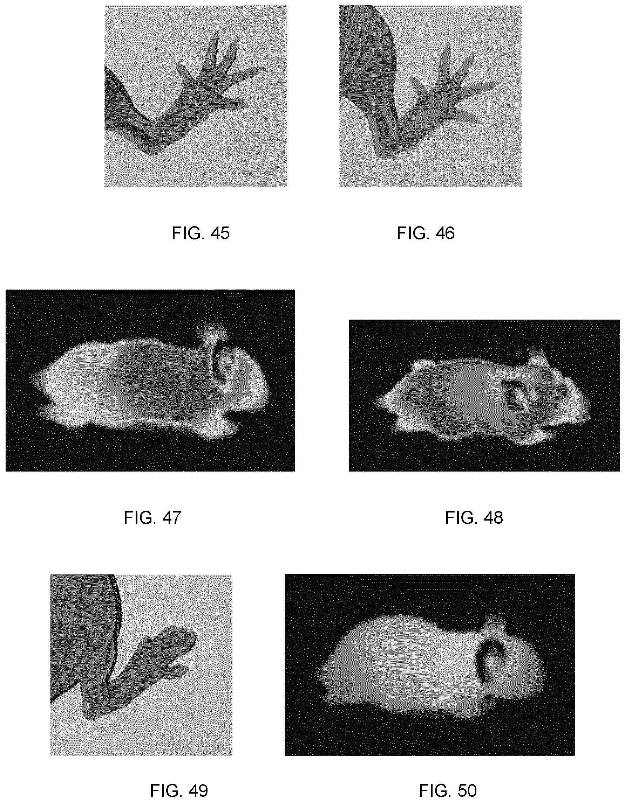

The mice in the target product group and the irradiation control group did not exhibit abnormal reactions after being irradiated with natural light. As shown in FIGs. 45 and 46, the limbs of the mouse were strong and the paw of the mouse was normal. As shown in FIGs. 47 and 48, there was no significant variation in the body temperature of the mouse.

The mouse in the reference compound group developed obvious photosensitization side effects after being irradiated by natural light, which was manifested by a decline in motor activity, muscle weakness, and obvious curling of the paw, as shown in FIG. 49. As shown in FIG. 50, a body temperature of the mouse was significantly decreased.

For the solvates of the porphyrin compounds such as water/ethanol/acetonitrile, the non-covalent bonded composites formed with proteins, and the prodrugs of the porphyrin compounds, after the same experimental verification as in Embodiments 6 - 12, it was found that these substances also have effects similar to the effects of the compound of Embodiment 3. For the compound with different substituents relative to Embodiment 3, an influence of the substituents on a peripheral benzene ring on an electronic structure of a macrocyclic main body may be neglected, the degradation process of the photosensitizer molecules may rely on lactone units in the structure reacting with the reactive oxygen species generated under irradiation. After the same experimental verification as in Embodiments 6-12, it was found that these substances also have effects similar to the substances of the compound of Embodiment 3.

In conclusion: the degradable near-infrared photosensitizer of the present disclosure has an absorption spectrum in the near-infrared region and a large extinction coefficient. Relative to the reference compound, the degradable near-infrared photosensitizer may be degraded while generating the reactive oxygen species in irradiation, after the irradiation is ended, the degradable near-infrared photosensitizer may be completely converted into a low-toxicity substance without photoactive. Therefore, the degradable near-infrared photosensitizer of the present disclosure may embody high phototoxicity in both the cells and the living body. The degradable near-infrared photosensitizer includes a relatively strong fluorescence, which may be configured for afluorescence labeling, and real-time monitoring of a degree of self-degradation.

The foregoing is only a preferred embodiment of the present disclosure and is not intended to limit the present disclosure, and any modifications, equivalent substitutions, improvements, etc. made within the spirit and principles of the present disclosure shall be included in the scope of protection of the present disclosure.

## Claims

1. A degradable near-infrared photosensitizer, wherein the degradable near-infrared photosensitizer is any one of a porphyrin compound, and a pharmaceutically acceptable salt, a solvate, a non-covalent bonded composite, a complex, and a prodrug of the porphyrin compound, and a structure of the porphyrin compound is as shown in formula 1: 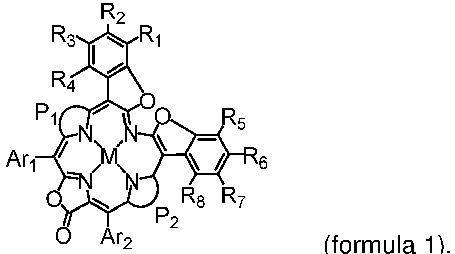 wherein 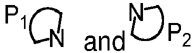 are independently selected from any one of 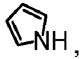 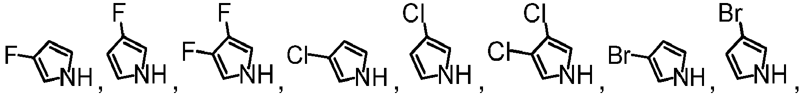 and 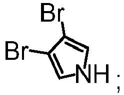
Ari and Ar₂ are substituted or unsubstituted phenyl; and
R₁, R₂, R₃, R₄, R₅, R₆, R₇, and R₈ are independently selected from any one of hydrogen, halogen, nitro, hydroxyl, amino, hydroxy, carboxy, carboxylic, sulfonic, phosphoric acid, cyano, amido, C₁-C₈ alkyl substituted amino, substituted or unsubstituted C₁-C₈ alkyl, substituted or unsubstituted C₁-C₈ alkoxy, substituted thio, C₁-C₈ alkyl phosphoryl, C₁-C₈ alkyl carboxy, C₁-C₈ alkyl sulfonyl, C₃-C₆ alkylene, C₂-C₆ alkenyl, C₂-C₆ alkinyl, C₂-C₆ alkynyl, C₂-C₆ alkenyloxy, and C₂-C₆ alkynyloxy, and M is a metal ion selected from any one of Mg, Cr, Mn, Fe, Co, Ni, Cu, Zn, Cd, Ga, Al, Pd, and Pt.

2. The degradable near-infrared photosensitizer of claim 1, wherein Ari and Ar₂ are independent 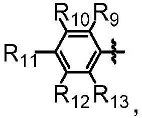 R₉, R₁₀, R₁₁, R₁₂, and R₁₃ are independently selected from any one of hydrogen, halogen, nitro, hydroxyl, amino, hydroxy, carboxy, carboxylic, sulfonic, phosphoric acid, cyano, amido, C₁-C₈ alkyl substituted amino, substituted or unsubstituted C₁-C₈ alkyl, substituted or unsubstituted C₁-C₈ alkoxy, substituted thio, C₁-C₈ alkyl phosphoryl, C₁-C₈ alkyl carboxy, C₁-C₈ alkyl sulfonyl, C₃-C₆ alkylene, C₂-C₆ alkenyl, C₂-C₆ alkinyl, C₂-C₆ alkynyl, C₂-C₆ alkenyloxy, and C₂-C₆ alkynyloxy.

3. The degradable near-infrared photosensitizer of claim 1, wherein the metal ion is any one of Mg, Zn, Cd, Al, and Ga.

4. The degradable near-infrared photosensitizer of any one of claims 1-3, wherein a specific structure of the porphyrin compound is any one of:

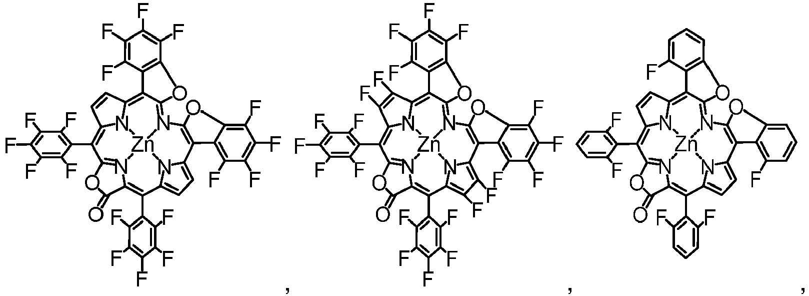

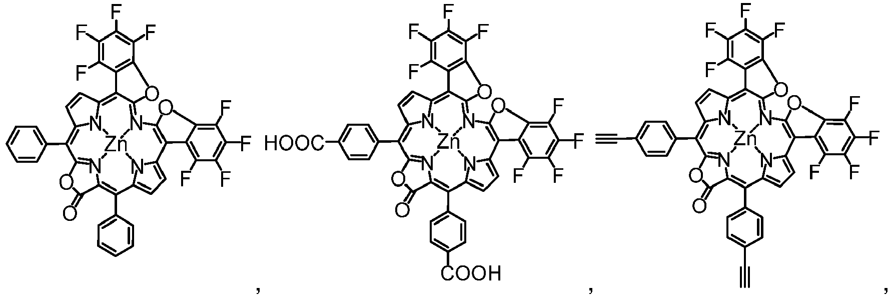

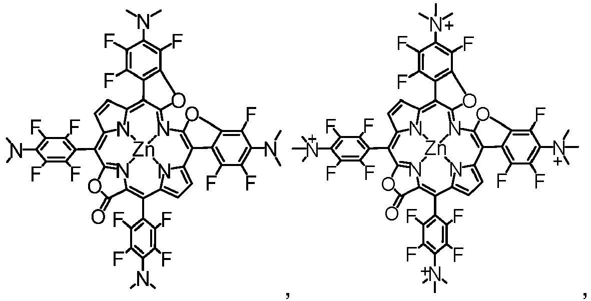

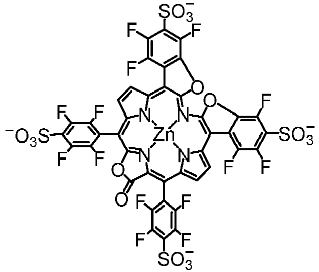

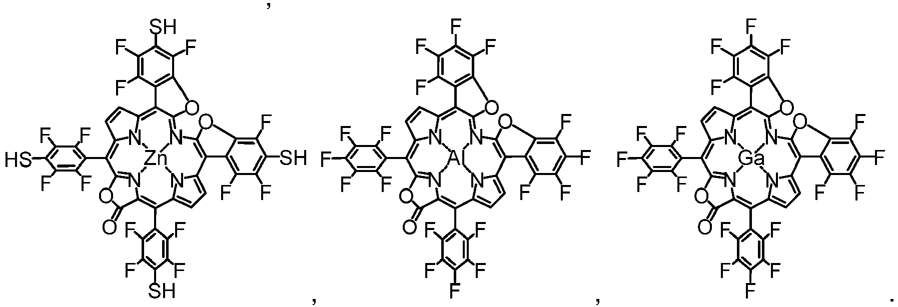

5. A preparation method for the degradable near-infrared photosensitizer of any one of claims 1-4, comprising:
operation 1: preparing a first intermediate product, including:
in a hydrothermal vessel, dissolving 100 mg of porphine dilactone and a base equivalent to 30 - 50 times the porphine dilactone in a range of 5 mL - 20 mL of a first solvent, and reacting at a range of 80 °C - 200 °C for a range of 24 h -72 h to obtain the first intermediate product, wherein a reaction equation of the operation 1 is: 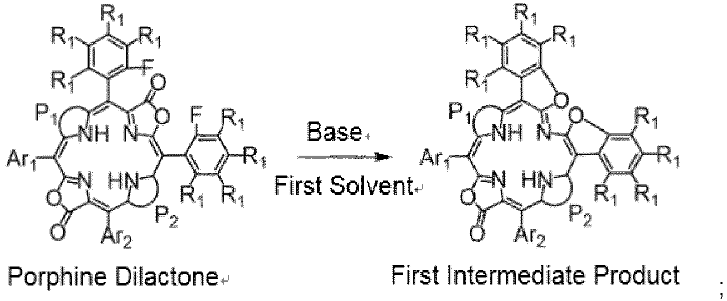
operation 2: preparing a second intermediate product, including:
performing a water-soluble modification reaction on 100mg of the first intermediate product obtained in operation 1 in a range of 5 mL - 50 mL of a second solvent to obtain the second intermediate product, wherein a reaction equation of the operation 2 is: 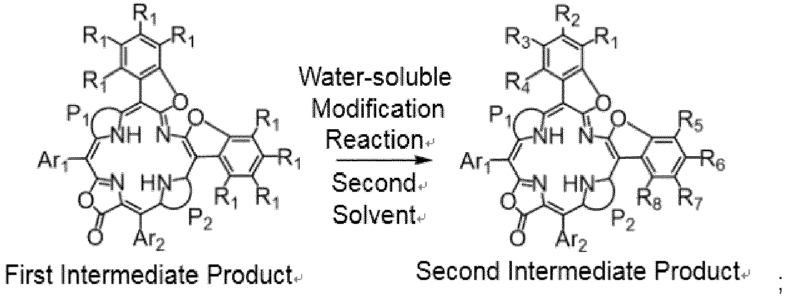
operation 3: preparing a target product, including:
dissolving 100 mg of the second intermediate product obtained in operation 2 and a metal salt equivalent to 10 times the second intermediate product in a range of 20 mL - 100 mL of a third solvent, reacting at a range of 20 °C - 80 °C for a range of 2 h - 16 h to obtain the target product shown in formula 1, wherein a reaction equation of the operation 3 is:

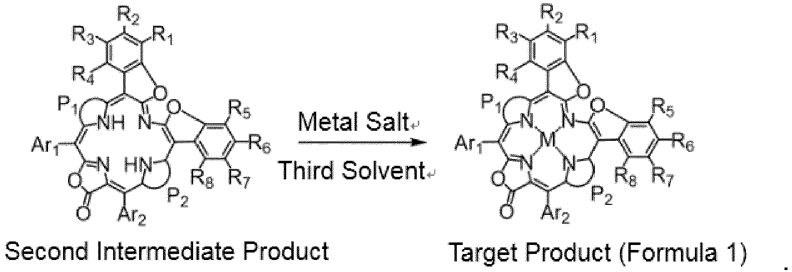

6. The preparation method for the degradable near-infrared photosensitizer of claim 5, wherein:
in operation 1, the base is any one or a mixture of two or more of sodium hydroxide, potassium hydroxide, potassium carbonate, sodium carbonate, triethylamine, N, N-dimethylformamide, sodium methanol, and potassium tert-butoxide;
the first solvent is any one or a mixture of two or more of water, methanol, ethanol, acetonitrile, acetone, dimethyl sulfoxide, N, N-dimethylformamide, and tetrahydrofuran; and
a temperature of the reaction in operation 1 is a range of 100 °C - 150 °C, and a time of the reaction in operation 1 is in a range of 48 h - 72 h.

7. The preparation method for the degradable near-infrared photosensitizer of claim 5, wherein the second solvent is any one or a mixture of two or more of dimethyl sulfoxide, N, N-dimethylformamide, tetrahydrofuran, ethyl acetate, trimethyl phosphate, triethyl phosphate, and hexanol.

8. The preparation method for the degradable near-infrared photosensitizer of claim 5, wherein:
the metal salt is any one of a magnesium salt, a chromium salt, a manganese salt, an iron salt, a cobalt salt, a nickel salt, a copper salt, a zinc salt, a cadmium salt, a gallium salt, an aluminum salt, a palladium salt, and a lead salt;
the third solvent is any one or a mixture of two or more of water, methanol, ethanol, acetonitrile, acetone, dimethylsulfoxide, N, N-dimethylformamide, tetrahydrofuran, trichloromethane, and methylene chloride; and
and a temperature of the reaction in operation 3 is in a range of 20 °C - 60 °C, and a time of the reaction in operation 3 is in a range of 3 h - 5 h.

9. The preparation method for the degradable near-infrared photosensitizer of claim 8, wherein the metal salt is any one of a magnesium salt, a zinc salt, a cadmium salt, a gallium salt, and an aluminum salt.

10. An application of the degradable near-infrared photosensitizer of any one of claims 1-4 as an active ingredient in a preparation of a photodynamic therapy medicine.
